# EUROPÄISCHE PATENTSCHRIFT

(11) **EP 0 776 202 B1**
(45) Veröffentlichungstag und Bekanntmachung des Hinweises auf die Patenterteilung: **17.05.2000**
(21) Anmeldenummer: 95929002.4
(22) Anmeldetag: 18.08.1995
(51) Int. Cl.: A61K 31/335, A61K 9/127

(54) **LIPOSOMAL VERKAPSELTES TAXOL, SEINE HERSTELLUNG UND SEINE VERWENDUNG**
LIPOSOME-ENCAPSULATED TAXOL, ITS PREPARATION AND ITS USE
TAXOL ENCAPSULE DANS DES PREPARATIONS LIPOSOMALES, SA PREPARATION ET SON UTILISATION

(30) Priorität: 20.08.1994 DE 4430593
(43) Veröffentlichungstag der Anmeldung: 04.06.1997
(73) Patentinhaber: MAX-DELBRÜCK-CENTRUM FÜR MOLEKULARE MEDIZIN, D-13125 Berlin (DE)
(72) Erfinder: RESZKA, Regine, D-16341 Schwanebeck (DE); BRANDL, Martin, D-79102 Freiburg (DE); FICHTNER, Iduna, D-13125 Berlin (DE); WARNKE, Gernot, D-79102 Freiburg (DE)
(74) Vertreter: Baumbach, Friedrich, Dr.
(86) Internationale Anmeldenummer: DE9501163
(87) Internationale Veröffentlichungsnummer: WO9605821

(56) Entgegenhaltungen:
- WO-A-93/18751
- WO-A-93/25225
- WO-A-94/26254
- US-A- 5 415 869
- CHEMICAL ABSTRACTS, vol. 121, no. 4, 25.Juli 1994 Columbus, Ohio, US; abstract no. 42658, & PHARM. RES., Bd. 11, Nr. 6, 1994 Seiten 889-896, A. SHARMA ET AL. 'NOVEL TAXOL FORMULATIONS:PREPARATION AND CHARACTERIZATION OF TAXOL-CONTAINING LIPOSOMES.'

## Beschreibung

Die Erfindung betrifft liposomal verkapseltes Taxol, seine Herstellung und seine Verwendung. Anwendungsgebiete der Erfindung sind die Medizin und die pharmazeutische Industrie.

Taxol ist ein in der Rinde verschiedener Eibenarten (Taxazeen) vorkommender Naturstoff, der aus diesen Rinden [J. Amer. Chem. Soc., 93:2325 (1971)] bzw. neuerdings auch durch chemische Synthese [J. Amer. Chem. Soc., 1110:5917-5919 (1988)] gewonnen werden kann. Taxol verfügt im Vergleich zu bisher bekannten Zytostatika über einen völlig neuartigen Wirkmechanismus [Ann. NY. Acad. Sci. 466:733-744 (1986); Sartorelli, A. (ed.): Molecular Actions and Targets for Cancer Chemotherapeutic Agents. Academic Press, New York, 1981, pp. 483-507]. Die Substanz fördert die Polymerisation der Mikrotubuli aus Tubulindimeren und stabilisiert die Mikrotubuli durch Hemmung ihrer Depolymerisation. Weiterhin kommt es zu einer abnormen Anordnung und Bündelung von Mikrotubuli während des gesamten Zellzykluses, was zur Bildung multipler mikrotubulärer Teilungssterne während der Mitose und damit zur Hemmung der normalen dynamischen Reorganisation des mikrotubulären Netzwerkes führt. Da dadurch die vitale Zellfunktion in der Interphase und während der Mitose entscheidend beeinflußt wird, zeigt Taxol eine deutliche antineoplastische Aktivität gegen verschiedene Tumoren, u.a. gegen implantierte B16-Melanome, P388-Leukämie und gegen humane Mammatumoren.
Die Anwendbarkeit von Taxol ist durch seine geringe Wasserlöslichkeit stark eingeschränkt. Lösungsvermittler wie Cremophor (polyethoxyliertes Castoröl) und Alkohol verbessern die Löslichkeit, führen jedoch bei der Anwendung zu erheblichen Nebenwirkungen, z.B. zu anaphylaktoiden Reaktionen. Die Verdünnung solcher Lösungen mit physiologischer Kochsalzlösung zur Applikation hat den Nachteil, daß Taxol in physiologischer Kochsalzlösung keine ausreichende Stabilität (maximal 24 Stunden) besitzt. Eine dosislimitierende Nebenwirkung ist die Myelosuppression in erster Linie die Neutropenie [Semin. Oncol. 19:646-662 (1992)]. Liposomen bieten aufgrund ihres amphiphilen Charakters die Möglichkeit sowohl wasser- als auch lipidlösliche Substanzen einzuschließen bzw. zu inkorporieren.
Taxol als eine fast wasserunlösliche Substanz läßt sich mit hoher Effizienz in der Lipidphase von Liposomen geeigneter Zusammensetzung lösen, so daß die durch Cremophor EL beim Menschen beobachteten Toxizitäten einschließlich einer vermehrten Chylomikronenbildung nicht mehr auftreten sollte. Die therapeutische Effektivität von freiem, liposomal verkapseltem und nanopartikulärem Taxol werden an zwei Leukämien, der P388 und der L1210, in vitro verglichen. Während die Wachstumshemmung der P388-Zellen für alle 3 Arzneimittelformen gleich war, zeigte die L1210 eine größere Sensitivität gegenüber der nanopartikulären Form.
Bei Testung aller 3 Zubereitungen an der P388 in vivo (12,5 mg/kg/4 Tage) waren die freie und liposomale Form in ihrer therapeutischen Effektivität vergleichbar, während nanopartikuläres Taxol akute Toxizitäten hervorrief [J. Microencapsul. 7 (2):191-7 (1990]. In einer weiteren Studie wurde Taxol in freier und liposomaler Form hinsichtlich der Antitumoraktivität an zwei humanen Glioblastomen im Nude-Maus-Modell getestet (12,5 mg/kg/4 Tage). Beide Formen führten zu einer signifikanten Verringerung des
Tumorwachstums [In-Vivo 6 (1):23-7 (992)]. Sharma et al. [Cancer Res. 53:5877-81 (1993)] und Straubinger et al. [J. Natl. Cancer Inst. Monographs 15:69-78 (1993)] berichten von einem signifikanten Antitumoreffekt von liposomalem Taxol (10-45 mg/kg) am murinen Taxol-resistenten Colon-26-Modell. In WO 93/18751 wird die Verkapselung von Taxol in Liposomen und die Verwendung der erhaltenen Produkte zur Behandlung von Krebserkrankungen beschrieben. Bevorzugt wird eine Kombination dieser Behandlung mit Hyperthermie. Die hergestellten Taxol-Liposomen zeigen eine verbesserte Stabilität.
Bisher wurde ein Patent (WO 93/18751) angemeldet, in dem eine Vielzahl von Taxol-Liposomen-Kombinationen beansprucht wird, von denen insbesondere Vesikel mit positiver Ladung auf der Basis von Cardiolipin, Phosphatidylcholin und Cholesterol hergestellt und untersucht wurden.
Das auf diese Weise verkapselte Taxol mußte jedoch in den Tierversuchen an vier aufeinanderfolgenden Tagen appliziert werden, da eine einmalige Gabe offensichtlich nicht zum gewünschten Antitumoreffekt führte.

Die Erfindung hat das Ziel, liposomal verkapseltes Taxol mit hohem Taxolanteil und höherer Stabilität, damit erhöhter therapeutischer Wirksamkeit, zur Verfügung zu stellen. Die Aufgabe besteht darin, spezifische Formen der Verkapselung von Taxol zu entwickeln und diese, ggf. in Kombination mit anderen freien bzw. liposomal verkapselten Substanzen, zur Behandlung unterschiedlicher Tumorarten und Lokalisationen einzusetzen. Im Vordergrund steht hierbei neben der möglichst einmaligen Applikation des liposomalen Taxols auch die Verhinderung des dosislimitierenden neutropenischen Effektes des unverkapselten Taxols z.B. durch Kombination mit liposomalem Carboplatin.
Die Aufgabe wird gemäß den Ansprüchen 1, 13, 14 und 16 gelöst, die Unteransprüche sind Vorzugsvarianten. Die Verkapselung wird erfindungsgemäß mittels Hochdruckhomogenisierung bzw. mittels Aerosolbildung durchgeführt.
Unsere eigenen Experimente umfassen Arbeiten zur Präparation und Charakterisierung verschiedener taxolhaltiger Liposomentypen unterschiedlicher Größe und Zusammensetzung (vgl. Ansprüche).

Gegenstand der Erfindung ist auch eine pharmazeutische Zubereitung, die das erfindungsgemäße, liposomal verkapselte Taxol und pharmazeutisch übliche Träger- und Zusatzstoffe enthält. Eine praktisch vorteilhaft einsetzbare Mischung enthält 0.98 mg Taxol in 50 mg Phosphatidylcholin (98% der eingesetzten Taxolmenge).
Die liposomale Verkapselung erfolgt durch Hochdruckhomogenisierung oder durch spontane Vesikelbildung. Im ersten Falle wird eine vorgefertigte Liposomen-Mischung in fester bzw. flüssiger Form mit Taxol vereinigt, nachfolgend mehrfach (bevorzugt 2 fach) bei 5-160 MPa homogenisiert und ggf. nachfolgend lyophilisiert.
Das Herstellungsverfahren zur Erzeugung einer Taxol-haltigen liposomalen Zubereitung mittels Hochdruckhomogenisation, sowie ggf. Freisetzung von Liposomen aus einer solchen Zubereitung umfaßt folgende Schritte:

### 1) Fakultativ Herstellung eines Lipidfilms und Überführung desselben in eine

Vormischung bestehend aus Membranbildnern und wäßrigem Medium: Herstellung eines dünnen, trockenen Lipidfilms durch Auflösen des Taxols zusammen mit den Membranbildnern in organischen Lösungsmitteln. Nachfolgend vollständige Entfernung der Lösungsmittel durch Evaporation ggf. durch Sprühtrocknung. Dispergieren des Lipidfilms nach Zugabe von wäßrigem Medium, z.B. durch Schütteln, Rühren, Kneten o.ä. ggf. unter erhöhter Temperatur (ca. 10 °C oberhalb der Phasenübergangstemperatur der Phospholipide).

### 2) Hochdruckhomogenisation:

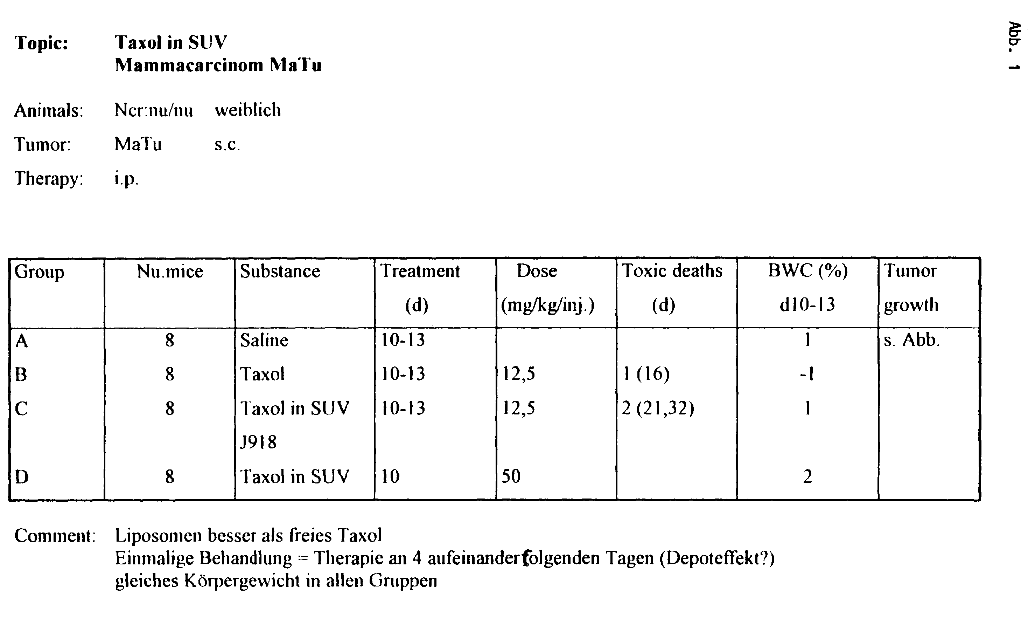

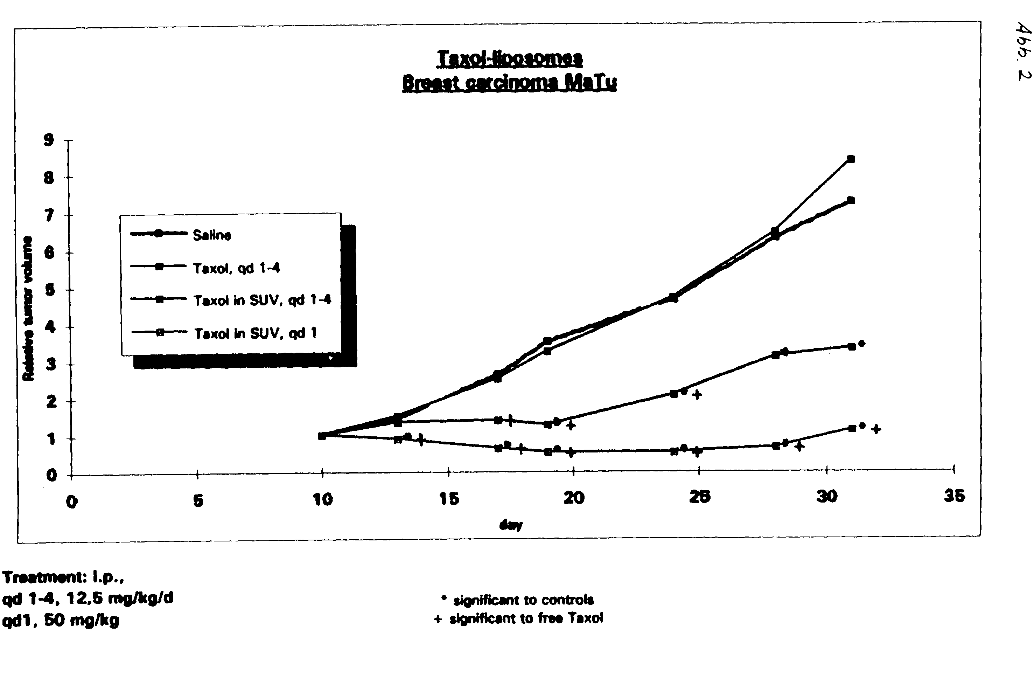

Entweder die aus 1) erhaltene Vormischung oder alternativ ein Gemisch aus Lipid(en), Wasser und Taxol (ggf. grob dispergiert durch Rühren z.B. mittels eines Magnetrührers o.ä.) wird ein- oder mehrmals (jedoch nicht mehr als fünfzigmal) der Hochdruckhomogenisation (z.B. mittels APV Gaulin Micron Für die tierexperimentelle Prüfung wurden Liposomen nach Beispiel 1 eingesetzt. Es konnte u.a. an einem menschlichen Mamma-Karzinom (MaTu) gezeigt werden, daß diese Liposomenpräparation im Vergleich zum freien Taxol (4 Tage Therapie, je Tag 12,5 mg/kg) bereits nach einmaliger Applikation von 50 mg/kg eine verbesserte therapeutische Wirksamkeit bei geringerer Hämatotoxizität aufwies (Abb. 1+2).

Als Verkapselungsmittel werden verwendet
a) ein natürliches, halbsynthetisches oder vollsynthetisches Amphiphil, wie ein Lipid, ein Tensid oder einen Emulgator,
b) eine geladene Lipidkomponente und/oder
   eine gesättigte Lipidkomponente,
   eine Etherlipidkomponente,
c) ein Polymer
d) eine Trägerflüssigkeit und ggf. zusätzliche Hilfsstoffe, wie z.B. Nano partikel enthält.

Das Amphiphil hat bevorzugt die in Anspruch 3 angegebene Formel I. Als geladene Lipidkomponente werden bevorzugt eingesetzt: das Anion des Dicethylphosphats, der Palmitinsäure, der Stearinsäure, das Anion eines Phospholipids, wie Phosphatidylserin oder Phosphatidsäure oder das Anion eines Sphingolipids wie Sulfatid. In einer besonders günstigen Ausführungsform der Erfindung wird als geladene Lipidkomponente Phosphatidylglycerol eingesetzt.
Die Erfindung kann gleichermaßen mit neutralen Lipidkomponenten wie Phosphatidylcholin oder gesättigten Lipidkomponenten wie Dipalmitoylphosphatidylcholin realisiert werden.
Als Polymer dient z.B. Polyethylenglycol vom Molekulargewicht 2000-10000. Als Trägerflüssigkeit wird im allgemeinen physiologische Kochsalzlösung eingesetzt. Lab) unter Drucken zwischen 5 und 160 Mpa (50 bis 1600 bar) unterzogen. Edukt, Homogenisator und Produkt werden ggf. temperiert.

### 3) Fakultativ Gefrier/Tau-Behandlung:

Durch ein- bis mehrmaliges Einfrieren und nachfolgendes Wiederauftauen der Zubereitung während, d.h. zwischen einzelnen Zyklen der Hochdruckhomogenisation oder an deren Ende wird eine homogenere Durchmischung des Ansatzes erreicht und evtl. die vorgebildeten Liposomen vorübergehend destabilisiert, so daß Liposomen mit noch mehr inkorporiertem Taxol gebildet werden.

### 4) Fakultativ Gefriertrocknen und Redispergieren:

Durch ein- bis mehrmaliges Gefriertrocknen und nachfolgendes Redispergieren der Zubereitungen bevorzugt während, d.h. zwischen einzelnen Zyklen der Hochdruckhomogenisation oder an deren Ende wird eine homogenere Durchmischung des Ansatzes erreicht und die vorgebildeten Liposomen vorübergehend destabilisiert, so daß Liposomen mit noch mehr inkorporiertem Taxol gebildet werden.

### 5) Fakultativ Überführung der Zubereitung in eine freifließende Liposomendispersion:

Durch schrittweise Zugabe von wäßrigem Medium in Volumenanteilen von 1 bis 30 % des Volumens der Zubereitung und mechanisches Mischen z.B. durch manuelles Schütteln oder mittels eines Vortex-Mischers wird das dreidimensionale Gelgerüst zerlegt und die vorgebildeten Liposomen freigesetzt.

### 6) Fakultativ Filtration der Liposomendispersion:

Falls die Liposomengröße nach oben hin begrenzt werden soll bzw. eine sterile Zubereitung erhalten werden soll, kann die Liposomendispersion durch Filter mit einer Porenweite von 0,1 µm bis 1 µm filtriert werden.

Die erfindungsgemäße Zubereitung, die Liposomen in dichter Packung enthält (Liposomengel), eignet sich als Wirkstoffdepot, das den Wirkstoff langsam freisetzt und zwar entweder in gelöster Form oder in Form einzelner Wirkstoff-enthaltender Liposomen. Diese Zubereitung kann durch Injektion (z.B. i.m., i.p.) oder Implantation angewendet werden. Aber auch ein Einbringen in Körperhöhlen oder ein Aufbringen auf Schleimhäute, auf die Hornhaut des Auges (Kornea) oder Hautpartien ist möglich. Somit dient die Zubereitung als Träger des Wirkstoffs sowie ggf. zu dessen modifizierter Freigabe.
Die erfindungsgemäße Zubereitung, die Liposomen in freifließender Form enthält, eignet sich als Träger des Wirkstoffs. Diese Zubereitung kann durch Injektion (z.B. i.v., i.m.) oder ebenfalls durch Einbringen in Körperhöhlen oder Aufbringen auf Schleimhäute, auf die Hornhaut des Auges (Kornea) oder Hautpartien angewendet werden. Die erfindungsgemäß verkapselten Liposomen führen zu einer Verteilung des liposomalen Wirkstoffs im Körper, die selektiv eine hohe und lange andauernde Wirkstoffkonzentration am Wirkort bewirkt und damit zu einer Verbesserung der Wirkung oder zu einer Verbesserung des Verhältnisses von Wirkung zu Nebenwirkung.

Zur spontanen Vesikelbildung wird die Taxol/Verkapselungsmittel/Treibgas-Mischung aus Dosieraerosolen versprüht und bildet nach dem Verdampfen des Treibgases auf der Lungenoberfläche spontan liposomal verkapseltes Taxol. Alternativ können vorgebildete Taxol-Liposomen vernebelt werden. Dieser Weg ist vor allem dann vorzuziehen, wenn ein direkter Einsatz gegen Lungenmetastasen beabsichtigt ist.
Das liposomal verkapselte Taxol ist als Zytostatikum verwendbar. Diese Verwendung kann allein oder in Kombination mit anderen Wirkstoffen wie Carboplatin oder liposomal verkapseltem Carboplatin erfolgen. Bevorzugt ist der Einsatz gegen Hirntumoren, maligne Melanome, Lebermetastasen, Lungenmetastasen, Mammakarzinome und gegen Urogenitalkarzinome.
Bei der Behandlung u.a. eines menschlichen Mamma-Karzinoms auf der Nude-Maus wurde nach einmaliger Gabe von 50 mg/kg liposomal verkapseltem Taxols, im Vergleich zur behandelten Taxolgruppe (4x12,5 mg/kg) eine signifikante Tumorvolumenhemmung erreicht.

Die Erfindung soll nachfolgend durch Ausführungsbeispiele näher erläutert werden.

### Beispiel 1

Ein Lipidfilm, bestehend aus 1500 mg Eiphosphatidylcholin und 30 mg Taxol, wird in 30 ml steriler, Calcium-freier Phosphat-gepufferter (pH 7,2-7,4) Kochsalzlösung dispergiert.
Die entstandene Dispersion multischichtiger Liposomen wird im Hochdruckhomogenisator (Gaulin Mikrolab 40) bei 700 bar behandelt. Die entstandene Liposomendispersion (SUV) ist bei 4 Grad Celsius kurzzeitig lagerfähig und eignet sich zur parenteralen (i.v.) Applikation.
Zur längeren Lagerung eignet sich die Gelbildung bzw. die Lyophilisierung.

### Beispiel 2

Liposomen, hergestellt wie im Beispiel 1 werden mit einem geeigneten Vernebler aerosolisiert (z.B. Aero-Tech II) und somit der inhalativen Applikation zugänglich gemacht.

### Beispiel 3

200 mg Taxol, 200 mg Verkapselungsmittel nach Anspruch 2 und 400 µl Ethanol (90%) werden in einen Druckgasbehälter dosiert. Nach Aufbördeln eines Dosierventils (z. B. Perfect Valois DF 10/150 ACT) wird die Zubereitung mit 10 ml druckverflüssigtem Dimethylether versetzt, so daß die klare bis opaleszierende oder leicht getrübte Lösung entsteht.

### Beispiel 4

Wie Beispiel 3, jedoch unter Verwendung von druckverflüssigtem Isobutan als Treibgas.

### Beispiel 5

Wie Beispiel 3, jedoch unter Verwendung von druckverflüssigtem Propan/Butan (15/85) als Treibgas.

## Patentansprüche

1. Verfahren zur Herstellung einer pharmazeutischen Zubereitung, enthaltend liposomal verkapseltes Taxol, dadurch gekennzeichnet, daß ein Gemisch von membranbildenden Amphiphilen, in denen Taxol gelöst wurde, und eine wässrige Phase, ggf. nach Herstellung eines dünnen, trockenen Lipidfilms, nachfolgender Entfernung der Lösungsmittel durch Evaporation und Dispergieren in Wasser, ein- oder mehrmals, höchstens fünfzigmal, einer Hochdruckhomogenisation mit Drucken von 50 bis 1600 bar (5-160 Mpa) unterzogen wird und ggf. nachfolgend eine Gefrier/Tau-Behandlung, ggf. eine Überführung in eine freifließende Dispersion und anschließend ggf. eine Extrusion durch Filter mit einer Porenweite von 0,1 bis 1 µm erfolgt.

2. Verfahren zur Herstellung einer pharmazeutischen Zubereitung, enthaltend liposomal verkapseltes Taxol, dadurch gekennzeichnet, daß eine vorgefertigte Liposomen-Mischung in fester bzw. flüssiger Form mit Taxol vereinigt und nachfolgend in speziellen Aerosol-bildenden Vorrichtungen behandelt wird.

3. Verfahren zur Herstellung einer pharmazeutischen Zubereitung, enthaltend liposomal verkapselters Taxol, dadurch gekennzeichnet, daß Taxol und die Verkapselungsmittel in einem druckverflüssigten Treibgas gelöst und nach Verdampfen des Treibgases durch spontane Vesikelbildung auf dem Lungenephithel in liposomal verkapseltes Taxol überführt werden.

4. Verfahren nach den Ansprüchen 1, 2 und 3, dadurch gekennzeichnet, daß als Verkapselungsmittel
a) ein natürliches, halbsynthetisches oder vollsynthetisches Amphiphil, wie ein Lipid, ein Tensid oder ein Emulgator,
b) eine geladene Lipidkomponente und/oder eine gesättigte Lipidkomponente und/oder eine Etherlipidkomponente,
c) ein Polymer
d) eine Trägerflüssigkeit und ggf. zusätzliche Hilfsstoffe, wie z. B. Nanopartikel eingesetzt werden.

5. Verfahren nach Anspruch 1 bis 4, dadurch gekennzeichnet, daß ein natürliches, halbsynthetisches oder vollsynthetisches Amphiphil der allgemeinen Formel I, 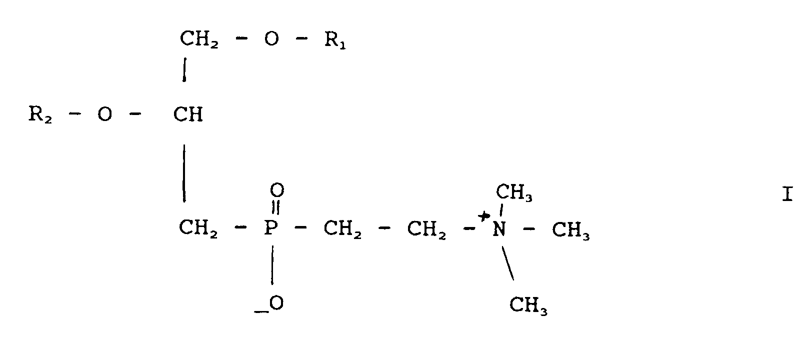 worin R₁ und R₂ C₁₀-C₂₀-Alkanoyl, -Alkenoyl, -Alkyl, -Alkenyl bedeuten, eingesetzt wird.

6. Verfahren nach Anspruch 4, dadurch gekennzeichnet, daß als geladene Lipidkomponente das Anion des Dicethylphosphats, der Palmitinsäure, der Stearinsäure, Etherlipide, das Anion eines Phospholipids, wie Phosphatidylserin oder Phosphatidylglycerol, Phosphatidsäure, oder das Anion eines Sphingolipids, wie Sulfatid oder ein chemisch modifiziertes Phosphatidylethanolamin, über das Proteine (wie z. B. Antikörper) angekoppelt werden können, eingesetzt wird.

7. Verfahren nach Anspruch 4, dadurch gekennzeichnet, daß als neutrale Lipidkomponente Phosphatidylcholin eingesetzt wird.

8. Verfahren nach Anspruch 4, dadurch gekennzeichnet, daß als gesättigte Lipidkomponenten Dipalmitoylphosphatidylcholin oder Dimyrestoylphosphatidylcholin eingesetzt wird.

9. Verfahren nach Anspruch 1 bis 4, dadurch gekennzeichnet, daß in bzw. an der Membran der Vesikel (über chemische Kopplung) Polyethylenglycol (MG 2000 - 10000) enthalten sind.

10. Verwendung von liposomal verkapseltem Taxol nach Anspruch 1 bis 9 gegebenenfalls in Kombination mit anderen Wirkstoffen zur Herstellung eines cytostatischen Arzneimittels.

11. Verwendung nach Anspruch 10 in Kombination mit liposomal verkapseltem Carboplatin oder in Kombination mit Carboplatin.

12. Verwendung von liposomal verkapseltem Taxol nach Anspruch 10 zur Herstellung eines Arzneimittels gegen Hirntumoren, gegen maligne Melanome, gegen Lebermetastasen, gegen Lungenmetastasen, gegen Mammakarzinome oder gegen Urogenitalkarzinome.

13. Verwendung von liposomal verkapseltem Taxol in Kombination mit liposomal verkapseltem Carboplatin in Aerosolform zur Herstellung eines Arzneimittels gegen Lungenmetastasen.

## Claims

1. A process for the manufacture of a pharmaceutical preparation containing liposomally encapsulated Taxol, characterised in that a mixture of membrane-forming amphiphils, in which Taxol was dissolved, and an aqueous phase, if necessary following production of a thin dry lipid film, subsequent separation of the solvent by means of evaporation and dispersion in water, once or a number of times, five times at most, is subjected to high-pressure homogenisation with pressures of 50 to 1600 bar (5 - 160 Mpa) and subsequently, if required, freezing / thawing treatment, if necessary conversion to a free-flowing dispersion and then, if required, extrusion through a filter having a porosity of 0.1 to 1 µm.

2. A process for the manufacture of a pharmaceutical preparation containing liposomally encapsulated Taxol, characterised in that a previously produced liposome mixture is combined in solid or liquid form with Taxol and subsequently treated in special aerosol-forming apparatus.

3. A process for the manufacture of a pharmaceutical preparation containing liposomally encapsulated Taxol, characterised in that Taxol and the encapsulating means are dissolved in a pressure-liquefied propellant and after vaporising of the propellant are converted into liposomally encapsulated Taxol by spontaneous vesicular development on the pulmonary epithelium.

4. A process as claimed in Claims 1, 2 and 3, characterised in that the following are used as encapsulating means:
a) a natural semi-synthetic or fully synthetic amphiphil, such as a lipid, a surfactant or an emulsifier,
b) a charged lipid component and/or
a saturated lipid component and/or
an ether lipid component,
c) a polymer,
d) a carrier liquid and, if required, additional adjuvants such as nano particles, for example.

5. A process as claimed in Claims 1 to 4, characterised in that a natural semi-synthetic or fully synthetic amphiphil is used of the formula 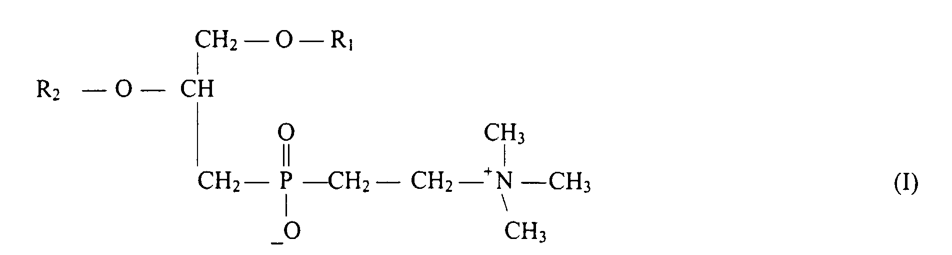 wherein R₁ and R₂ mean C₂₀-C₂₀-alkanoyl, -alkenoyl, -alkyl and -alkenyl.

6. A process as claimed in Claim 4, characterised in that the following are used as charged lipid components: the anion of dicethylphosphate, palmitin acid, stearic acid, ether lipids, the anion of a phospholipid such as phosphatidyl serin or phosphatidyl glycerin, phosphatide acid, or the anion of a sphingolipid such as sulfatide or a chemically modified phosphatidyl ethanolamine, by means of which proteins, such as antibodies, for example, can be coupled.

7. A process as claimed in Claim 4, characterised in that phosphatidyl choline is used as neutral lipid components.

8. A process as claimed in Claim 4, characterised in that dipalmitoylphosphatidyl choline or dimyrestoylphosphatidyl choline are used as saturated lipid components.

9. A process as claimed in Claims 1 to 4, characterised in that polyethylene glycol (MG 2000 - 10000) is contained in or on the membrane of the vesicle, by way of chemical coupling.

10. Use of liposomally encapsulated Taxol as claimed in Claims 1 to 9 in combination with other substances for the manufacture of an anti-tumor pharmaceutical , if required.

11. Use as claimed in Claim 10 in combination with liposomally encapsulated carboplatin or in combination with carboplatin.

12. Use of liposomally encapsulated Taxol as claimed in Claims 10 for the manufacture of a pharmaceutical to be used against brain tumors, malignant melanomas, liver metastases, lung metastases, breast carcinomas or against urogenital carcinomas.

13. Use of liposomally encapsulated Taxol in combination with liposomally encapsulated carboplatin in aerosol form for the manufacture of an anti-tumor pharmaceutical to be used against lung metastases.

## Revendications

1. Procédé pour la fabrication d'une préparation pharmaceutique contenant du taxol encapsulé dans des liposomes, caractérisé en ce qu'un mélange d'amphiphiles formateurs de membrane, dans lequel du taxol a été dissous, et d'une phase aqueuse est soumis une ou plusieurs fois, au maximum cinquante fois, éventuellement après création d'un mince film lipidique sec, élimination subséquente des solvants par évaporation et dispersion dans l'eau, à une homogénéisation sous haute pression à une pression de 50 à 1600 bar (5 à 160 mPa) et un traitement par congélation-dégel est éventuellement exécuté ensuite et éventuellement suivi d'une transformations en une dispersion fluide et éventuellement d'une extrusion à travers des filtres ayant une taille de pores de 0,1 à 1 µm.

2. Procédé pour la fabrication d'une préparation pharmaceutique contenant du taxol encapsulé dans des liposomes, caractérisé en ce qu'un mélange de liposomes déjà préparé sous forme liquide ou solide est mélangé avec le taxol puis traité dans des dispositifs spéciaux générant des aérosols.

3. Procédé pour la fabrication d'une préparation pharmaceutique contenant du taxol encapsulé dans des liposomes, caractérisé en ce que le taxol et les substances d'encapsulation sont dissous dans un gaz porteur liquéfié sous pression et transformés en taxol encapsulé dans des liposomes par la formation spontanée de vésicules sur l'épithélium pulmonaire après l'évaporation du gaz porteur.

4. Procédé selon les revendications 1, 2 et 3, caractérisé en ce que les substances d'encapsulation utilisés comprennent :
a) un amphiphile naturel, semi-synthétique ou de synthèse tel qu'un lipide, un agent tensioactif ou un émulsifiant,
b) une composante lipidique chargée et/ou
une composante lipidique saturée et/ou
une composante lipide-éther,
c) un polymère,
d) un liquide porteur et éventuellement des adjuvants supplémentaires, par exemple des nanoparticules.

5. Procédé selon les revendications 1 à 4, caractérisé en ce qu'il met en oeuvre un amphiphile naturel, semi-synthétique ou de synthèse selon la formule générale I, 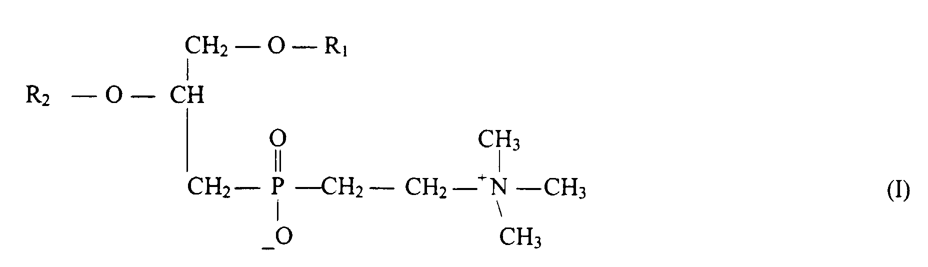 où R₁ et R₂ désignent un alcanoyle, un alkénoyle, un alkyle ou un alkényle C₁₀-C₂₀.

6. Procédé selon la revendication 4, caractérisé en ce qu'on utilise comme composante lipidique chargée l'anion du phosphate de dicéthyle, l'acide palmitique, l'acide stéarique, des lipides-éthers, l'anion d'un phospholipide tel que la phosphatidylsérine ou le phosphatidylglycérol, l'acide phosphatidique ou l'anion d'un sphingolipide tel que le sulfatide ou une phosphatidyléthanolamine modifiée chimiquement, qui permettent la liaison de protéines (par exemple d'anticorps).

7. Procédé selon la revendication 4, caractérisé en ce qu'on utilise comme composante lipidique neutre de la phosphatidylcholine.

8. Procédé selon la revendication 4, caractérisé en ce que l'on utilise comme composantes lipidiques saturées de la dipalmitoylphosphatidylcholine ou de la dimyristoylphosphatidylcholine.

9. Procédé selon les revendications 1 à 4, caractérisé en ce que du polyéthylène-glycol (PM 2000-10 000) est présent dans ou sur la membrane des vésicules (par liaison chimique).

10. Utilisation de taxol encapsulé dans des liposomes selon les revendications 1 à 9, éventuellement combiné à d'autres principes actifs, pour la fabrication d'un médicament cytostatique.

11. Utilisation selon la revendication 10, combiné à de la carboplatine encapsulée dans des liposomes ou à de la carboplatine.

12. Utilisation de taxol encapsulé dans des liposomes selon la revendication 10 pour la fabrication d'un médicament contre les tumeurs cérébrales, les mélanomes malins, les métastases hépatiques, les métastases pulmonaires, les cancers du sein et les cancers de l'appareil urogénital.

13. Utilisation de taxol encapsulé dans des liposomes en combinaison avec de la carboplatine encapsulée dans des liposomes, sous forme d'aérosol, pour la fabrication d'un médicament contre les métastases pulmonaires.
